# EUROPEAN PATENT SPECIFICATION

(11) **EP 2 802 307 B1**
(45) Date of publication and mention of the grant of the patent: **03.07.2019**
(21) Application number: 13700210.1
(22) Date of filing: 08.01.2013
(51) Int. Cl.: A61Q 19/00, A61Q 1/06, A61Q 15/00, A61K 8/02, A61K 8/19, A61K 8/81, B29K 91/00, B29L 31/00, B29C 39/42

(54) **SOLID COSMETIC COMPOSITION WITH MAGNETIC EFFECTS**
FESTE KOSMETISCHE ZUBEREITUNG MIT MAGNETISCHER WIRKUNGEN
COMPOSITION COSMÉTIQUE SOLIDE AVEC DES EFFETS MAGNÉTIQUES

(30) Priority: 10.01.2012 FR 1250242; 29.02.2012 US 201261604888 P
(43) Date of publication of application: 19.11.2014
(73) Proprietor: L'OREAL, 75008 Paris (FR)
(72) Inventor: PINHEIRO-BAIRRAS, Bernardino, F-77181 Courtry (FR)
(74) Representative: L'Oreal
(86) International application number: PCT/EP2013/050216
(87) International publication number: WO 2013/104619

(56) References cited:
- DE-B3-102006 008 077
- FR-A1- 2 888 115
- US-A1- 2006 088 483
- US-A1- 2007 148 105
- US-A1- 2008 292 862
- US-A1- 2009 185 992

## Description

The present invention relates to a process for manufacturing a solid cosmetic composition, especially for caring for and/or making up the skin, especially of the body, the hands, the neck, the face and the lips.

More particularly, the compositions under consideration according to the invention may constitute a body and/or lip makeup product especially having non-therapeutic care and/or treatment properties.

In general, these products contain, besides a fatty phase such as wax and/or oil, mineral fillers and/or pigments in variable proportions so as to give them a shade and/or various cosmetic properties. It is thus known practice to introduce fillers into this type of composition in order to adjust its properties in terms of texture and especially to thicken it. Similarly, fillers are generally incorporated into lipsticks to limit the migration of oils in the fine lines of the lips

Women use various makeup products to embellish their lips.

Lip glosses, lip pencils and lipsticks create advantageous shapes and volumes via gloss, colour and contrast effects. These expensive cosmetic wands involve combined applications and expert application actions.

Lip products combining a lipstick and a liner that simplify the application are known. The heart of the grape is composed of light-coloured lipstick and the contour, which acts as a liner, is composed of a dark colour. After depositing the product on the lips, the light and glossy colour is spread over the centre of the lips and the dark colour on the edge of the lips, generating a shadow that gives the illusion of more voluminous lips.

These products offer a cheap, simple and temporary means for creating fleshy lips.

These two-compound grapes take various forms and combine varied colours.

However, it is at the present time impossible to obtain lipsticks composed of complicated patterns, particularly aesthetic or novel patterns. The reason for this is that the lipsticks that are currently available comprising several visible phases are generally formed from a cylindrical core made with a first colour, this core being coated with a layer of another colour. Beyond this presentation, few choices are available to consumers.

In addition, non-fluid care or makeup products occasionally have the annoying tendency of having a fragile region at the interface. This is detrimentally perceived by the user, who observes that her two-phase lipstick wand, for example, breaks more easily than a standard single-colour wand. Thus, the coating may break gradually as the wand is consumed.

Moreover, the peripheral layer of certain sticks, known as the liner, is currently very thin on target parts of the grape. It follows that the deposit on the edge of the lips is coarse and not sufficiently visible. As such, the makeup result with relief and the desired effect is still not ideal.

In order to improve the aesthetic qualities of certain sticks, they have a bevelled shape. Nevertheless, it is at the present time impossible to produce all the bevels desired for two-phase sticks. Furthermore, the advantages of the starting product disappear gradually as the stick is worn down.

It has even been observed, with the current two-phase sticks, that there are adhesion defects between the two coloured layers and a risk of slippage which deteriorates the product.

There is thus a need to provide a solid composition for making up and/or caring for the skin and/or the lips that does not have the drawbacks mentioned above.

The present invention relates to a process for manufacturing a solid cosmetic composition cast as a stick the said composition having an outer surface, and containing one or more magnetic bodies (1) of non-zero magnetic susceptibility, the magnetic bodies (1) are on at least part of the said outer surface non-randomly oriented in the solid composition so as to form on the said outer surface one or more pattern(s) (5), the process is characterized in that it comprises the following steps:
- preparing a fluid cosmetic composition comprising one or more magnetic bodies (1) of non-zero magnetic susceptibility,
- casting the cosmetic composition while hot as a stick,
- at least partially exposing the composition to a magnetic field (10), so as to modify the orientation of and/or at least partly to move the magnetic bodies (1) when the composition is fluid, in order to distribute them non-randomly in the solid composition.

Preferably, the composition according to the invention is cast while hot, especially between 75°C and 120°C.

According to the invention, the magnetic bodies of non-zero magnetic susceptibility are in particular mobile due to the effect of a magnetic field when the composition is fluid.

As examples of known processes for manufacturing non-fluid compositions, document WO 02/036 992 discloses a lipstick obtained by moulding the core, solidifying it and then depositing a liquid composition onto the solidified core. A lipstick that accentuates the thickness of the lips is moreover described in WO 98/46436. This lipstick is manufactured in a mould comprising several movable partitions. The core is made first and solidifies in a first region of the mould having first movable partitions. These partitions are removed as soon as solidification is obtained. Another part is cast with another colour in a second region having second movable walls. Once this second part is cast, the partition is removed. A third and then a fourth part may also be made in this way.

Other methods known to those skilled in the art differ from double-casting, for instance spraying and printing. They also make it possible to produce colour patterns on the contour of makeup wands. However, they are neither precise nor efficient. In the case of compositions in the form of a stick of lipstick, the surface decorations are obtained by spraying on nacre. This type of decoration requires specific tooling, which remains expensive. Furthermore, with this method, the sticks are entirely covered with a thin film of nacre. As the nacre is retained to a greater or lesser extent by the stick, the production of a pattern is even more difficult.

There is thus a need to provide a process for manufacturing a solid composition for making up and/or caring for the skin and/or the lips that is simpler and less expensive than the above processes.

The magnetic bodies used in the process of the invention are mobile when the composition is in the fluid state.

When the magnetic bodies provide colour, a change in their orientation due to the effect of the magnetic field may lead to a change in the appearance of the composition.

According to the invention, the solid cosmetic composition manufactured is in stick form.

The process according to the invention applies to the existing solid cosmetic formulations. It does not involve any adaptation other than the addition of magnetic nacres, and thus preserves the properties of the formulations.

The lipsticks obtained via this process comprise variable colour intensities.

When the magnetic bodies are moved, the form of the composition may be thereby affected, which makes it possible, for example, to create a relief.

The magnetic field may be applied so as to form at least one pattern on the composition, this pattern being associated, for example, with the geometry of the field lines.

The process thus enables the creation of novel effects in a cosmetic composition, making it possible, for example, to produce patterns in relief or giving an impression of relief or various other geometrical or non-geometrical patterns.

In the process according to the invention, the magnetic field may be applied until a set appearance of the composition containing the magnetic bodies is obtained, i.e. until the composition ceases to change even if the magnetic field remains.

The magnetic bodies are mobile in the fluid composition. Their mobility and their speed of travel decrease when the composition sets to a solid. They are fixed in place when the composition is solidified.

Advantageously, the composition obtained by the process according to the invention has a melting point or a thermal transition temperature of greater than 25°C, especially ranging from 25 to 110°C and in particular ranging from 30 to 60°C and/or a hardness ranging from 0.001 to 0.5 MPa and especially ranging from 0.005 to 0.4 MPa. These melting point and hardness ranges have the advantage of leading to better migration of the magnetic bodies and to sharper patterns on stick.

More advantageously, the composition obtained by the process according to the invention is in the form of lipsticks, concealer products, complexion "correcting" or "embellishing" products, and/or deodorants or antiperspirants.

According to a first embodiment of the compositions according to the invention, they comprise one or more regions that are rich in magnetic bodies and one or more regions that are depleted in magnetic bodies. In particular, these regions may be visible to the eye and may form a more or less complicated decoration.

Preferentially, the regions form several mutually isolated patterns. The advantage of such a distribution of the patterns is that they may be constantly renewed gradually as the composition is worn down.

Even more preferentially, the pattern(s) are repetitive. They may thus serve as a marker of the degree of wear for the user, who can detect the total number of patterns before the first use and follow the state of progress of her composition.

Advantageously, the total concentration of magnetic bodies is between 0.2% and 10% by weight relative to the total weight of the composition, preferably between 0.3% and 5% and even more preferentially between 0.5% and 3%. With these concentration ranges, the magnetic bodies can migrate freely under the effect of the magnetic field and follow the morphology of the magnetic induction field, to form clear patterns.

More advantageously, the concentration of magnetic bodies in the regions rich in magnetic bodies is between 5% and 10% by weight relative to the total weight of the composition, preferably between 10% and 20% and even more preferentially between 15% and 25%.

Even more advantageously, the concentration of magnetic bodies in the regions depleted in magnetic bodies is between 1% and 0.5% by weight relative to the total weight of the composition, preferably between 0.5% and 0.02% and even more preferentially between 0.05% and 0.001%.

More advantageously, in this process, the cosmetic composition is cast at a temperature of between 75 and 120°C and more preferentially between 85 and 110°C.

The invention also relates to the solid compositions that may be obtained via the above process according to the invention.

### Solid composition

A cosmetic composition may be "solid" in accordance with the invention from the moment that it has a melting point or a thermal transition temperature such as the softening point of greater than 25°C, which may especially range from 25 to 85°C, or even from 30 to 60°C and in particular from 30 to 45°C and/or a hardness that may range from 0.001 to 0.5 MPa and especially from 0.005 to 0.4 MPa.

The hardness of a composition may be determined by measuring the compression force, measured at 20°C using the texturometer sold under the name TA-XT2i® by the company Rheo, equipped with a stainless-steel cylindrical spindle 2 mm in diameter, travelling at a measuring speed of 0.1 mm/second, and penetrating the composition to a penetration depth of 0.3 mm. The hardness value is the maximum compression force measured divided by the surface area of the texturometer cylinder in contact with the composition. In the particular case of lipsticks, the hardness may also be measured via the cheese-wire method, which consists in cutting a lipstick wand 8.1 mm in diameter and in measuring the hardness at 20°C using a DFGHS2 tensile testing machine from the company Indelco-Chatillon travelling at a speed of 100 mm/minute. The measured hardness is expressed as the shear force (expressed in gram-force) required to cut a stick under these conditions.

A product cast as a stick obtained by the process according to the invention may be chosen especially from lipsticks concealer products, complexion "correctors" and/or "embellishers", deodorants and antiperspirants.

### PHYSIOLOGICALLY ACCEPTABLE MEDIUM

The term "physiologically acceptable medium" denotes a non-toxic medium that may be applied to human skin or lips. The physiologically acceptable medium is generally suited to the nature of the support onto which the composition is to be applied, and also to the aspect in which the composition is intended to be conditioned.

### Magnetic device

The magnetic field is generated by a magnetic device. This device may comprise one or two permanent magnets or an electromagnet powered, for example, by at least one battery or accumulator. In the latter case, the magnetic device may comprise a switch for selectively supplying the electromagnet with electricity.

The magnetic device may be arranged to create a magnetic field whose orientation varies over time. When the magnetic device comprises a magnet, the device may, for example, comprise a motor for driving the magnet in rotation. As a variant, the magnetic device may comprise several solenoids arranged so as to generate, when supplied sequentially with electricity, a rotating magnetic field.

A rotating magnetic field may make it possible, for example, to obtain a pattern with rotational symmetry, for example a pattern giving the impression of a sphere in relief.

The electromagnet(s) may be powered permanently or intermittently, at the user's choice. In particular, the magnetic device may be arranged such that the electromagnet(s) are not powered as long as the magnetic device is not correctly positioned close to the support coated with the first composition.

The magnetic field is, for example, at least 50 mT, or even at least 66 mT, better still at least 0.2 T, or even at least 1 T (10 000 Gauss) or 1.4 T (14 000 Gauss).

In order to make the application of the magnetic field easier, the magnetic device may comprise a member for positioning it relative to the mould in which the composition has been cast. This may make it possible, for example, to prevent the magnetic device from accidentally coming into contact with the composition and/or to centre the pattern produced on the area concerned.

The magnetic field may also be exerted by means of a magnetic structure, which is especially flexible, comprising an alternation of N and S poles. Such a structure may make it possible, for example, to produce repeating patterns on the composition, for example stripes.

### Magnetic bodies

The term "magnetic bodies" should not be understood in a limiting manner, but covers particles, fibres or particle and/or fibre agglomerates, of any form, having a non-zero magnetic susceptibility.

The composition applied may comprise magnetic fibres or other aspherical bodies, such as particle or fibre chains.

Preferably, the magnetic bodies have no remanent magnetization in the absence of a magnetic field.

The magnetic bodies may comprise any magnetic material that is sensitive to the lines of a magnetic field, whether this field is produced by a permanent magnet or derived from an induction, this material being chosen, for example, from neodymium, boron, nickel, cobalt, iron, alloys and oxides thereof, especially Fe₃O₄, and also gadolinium, terbium, dysprosium or erbium, and alloys and oxides thereof. The magnetic material may be of "soft" or "hard" type. The magnetic material may especially be soft iron.

The magnetic bodies may or may not have a multilayer structure, comprising at least one layer of a magnetic material, for instance iron, nickel or cobalt, and alloys and oxides thereof, especially Fe₃O₄.

The magnetic bodies are preferably aspherical, for example having an elongated shape. Thus, when these bodies are subjected to a magnetic field, they become oriented with their longitudinal axis in the alignment of the field lines, and undergo a change of orientation that is reflected by a change in the appearance of the composition.

When the magnetic bodies are substantially spherical particles, they are preferably of non-uniform appearance, such that a change in orientation induces a change in appearance.

The dimensions of the bodies, whatever their shape, is, for example, between 1 nm and 10 mm, better still between 10 nm and 5 mm, even better still between 100 nm and 1 mm, for example between 0.5 µm and 300 µm or 1 µm and 150 µm. The size is the dimension given by the statistical distribution for half the population, referred to as D50.

When the bodies are particles that do not have an elongated shape or that have an elongated shape with a relatively small aspect ratio, the particle size is, for example, less than 1 mm.

The magnetic bodies are, for example, magnetic pigments.

### Magnetic pigments

Pigments that are most particularly suitable for use are nacres comprising iron oxide Fe₃O₄. Pigments with magnetic properties are, for example, those sold under the trade names Colorona Blackstar Blue, Colorona Blackstar Green, Colorona Blackstar Gold, Colorona Blackstar Red, Cloisonne Nu Antique Super Green, Microna Matte Black (17437), Mica Black (17260), Colorona Patina Silver (17289) and Colorona Patina Gold (117288) from the company Merck or Flamenco Twilight Red, Flamenco Twilight Green, Flamenco Twilight Gold, Flamenco Twilight Blue, Timica Nu Antique Silver 110 AB, Timica Nu Antique Gold 212 GB, Timica Nu-Antique Copper 340 AB, Timica Nu Antique Bronze 240 AB, Cloisonne Nu Antique Green 828 CB, Cloisonne Nu Antique Blue 626 CB, Gemtone Moonstone G 004, Cloisonne Nu Antique Red 424 CB, Chroma-Lite Black (4498), Cloisonne Nu Antique Rouge Flambé (code 440 XB), Cloisonne Nu Antique Bronze (240 XB), Cloisonne Nu Antique Gold (222 CB) and Cloisonne Nu Antique Copper (340 XB) from the company Engelhard.

As another example of a magnetic pigment that can be included in the formulation of the composition, mention may be made of black iron oxide particles, for example those sold under the name Sicovit Black E172 by the company BASF.

The magnetic pigments may also comprise iron metal, especially passivated soft iron, for example obtained from iron carbonyl by means of the process described in patent US 6 589 331. These particles may comprise a surface layer of an oxide.

Particles based on soft iron are sold especially under the name Stapa® WM Iron VP 041040 by the company Eckart.

### Magnetic fibres

The term "fibres" denotes generally elongated bodies, for example having an aspect ratio ranging from 3.5 to 2500 or from 5 to 500, for example from 5 to 150. The aspect ratio is defined as the ratio L/D, in which L is the length of the fibre and D is the diameter of the circle in which the largest cross section of the fibre is inscribed.

The cross section of the fibres may be inscribed, for example, in a circle with a diameter ranging from 2 nm to 500 µm, for example ranging from 100 nm to 100 µm or even from 1 µm to 50 µm.

The fibres may have, for example, a length ranging from 1 µm to 10 mm, for example from 0.1 mm to 5 mm or even from 0.3 mm to 3.5 mm.

The fibres may have a mass ranging, for example, from 0.15 to 30 denier (mass in grams per 9 km of yarn), for example from 0.18 to 18 denier.

The fibres may have any cross sectional shape, for example circular or polygonal, especially square, hexagonal or octagonal.

The composition may comprise solid or hollow fibres, which are independent or bound together, for example braided.

The composition may comprise fibres with blunted and/or rounded ends, for example by polishing.

The fibres may not have their form substantially modified when they are introduced into the composition, for example being initially rectilinear and rigid enough to conserve their form. As a variant, the fibres may have a flexibility that enables them to be substantially deformed within the composition.

The fibres may comprise a non-zero content, which may be up to 100%, of a magnetic material chosen from soft magnetic materials, hard magnetic materials, especially based on iron, zinc, nickel, cobalt or manganese, and alloys and oxides thereof, especially Fe₃O₄, rare-earth metals, barium sulfate, iron-silicon alloys, optionally doped with molybdenum Cu₂MnAl, MnBi, or a mixture thereof, this list not being limiting.

When the composition comprises fibres containing magnetic particles, these particles may be present, for example, at least at the surface of the fibre, or even only at the surface of the fibres, only inside the fibre, or may be dispersed substantially homogeneously within the fibre.

The fibres may comprise, for example, a non-magnetic core with a plurality of magnetic particles at its surface.

The fibres may also comprise a synthetic matrix containing a plurality of magnetic grains dispersed therein.

Where appropriate, a synthetic material doped with magnetic particles may itself be coated with a non-magnetic shell. Such a shell constitutes, for example, a barrier isolating the magnetic material(s) from the ambient medium and/or may introduce colour. The fibres may comprise a monolithic magnetic core and may be coated with a non-magnetic shell, or vice versa.

The composition may comprise fibres made by extrusion or coextrusion of one or more polymers, especially thermoplastics and/or elastomers. One of the extruded materials may contain a charge of dispersed magnetic particles.

The fibre may comprise a synthetic material chosen from polyamides, PET, acetates, polyolefins, especially PE or PP, PVC, polyester block amide, plasticized Rilsan®, elastomers, especially polyester elastomers, PE elastomers, silicone elastomers or nitrile elastomers, or a mixture of these materials, this list not being limiting.

The composition may contain composite fibres comprising a magnetic core at least partially coated with at least one synthetic or natural non-magnetic material. The coating of the magnetic core may be performed, for example, by coextrusion, around the core, of a shell made of a non-magnetic material.

The core may be coated by another means, for example by *in situ* polymerization.

The core may be monolithic or may comprise a charge of magnetic grains dispersed in a matrix.

The composition may also contain composite fibres obtained by the coating with a synthetic material, doped with magnetic particles, of a natural or synthetic non-magnetic core, the core being composed, for example, of a wood fibre, rayon fibre, polyamide fibre, fibre of a vegetable matter, polyolefin fibre, especially polyethylene fibre, Nylon® fibre, polyimide-amide fibre or aramid fibre, this list not being limiting.

The composition may also comprise magnetic composite particles, especially a magnetic latex.

### Magnetic composite particles

A magnetic composite particle is a composite particle consisting of an organic or mineral matrix and of magnetic grains. The magnetic composite particles may thus comprise grains of a magnetic material at their surface and/or inside them. The composite particles may consist of a magnetic core coated with an organic or mineral matrix, or vice versa.

The magnetic composite particles comprise, for example, one of the abovementioned magnetic materials.

The size of the magnetic composite particles is, for example, between 1 nm and 1 mm, better still between 100 nm and 500 µm and even better still between 500 nm and 100 µm. The term "size" denotes the dimension given by statistical particle size distribution for half the population, referred to as D50.

The thesis by C. Goubault, 23 March 2004, recalls in chapter 1 the state of the art regarding magnetic composite particles, and draws up a list of the preparation processes that may be used for preparing magnetic composite particles, namely a separate synthesis of the magnetic grains and of the matrix, a synthesis of the magnetic grains in contact with the matrix or a synthesis of the matrix in the presence of the magnetic grains.

The company Kisker markets composite magnetic particles with a mineral matrix, composed of silica. The companies Dynal, Seradyn, Estapor and Ademtech propose composite magnetic particles with an organic matrix, which may also be used in the invention.

More particularly, the company Estapor markets under the reference M1-070/60 two magnetic latices consisting of ferrite grains uniformly distributed in a polystyrene matrix, this latex comprising 65% iron oxide, the mean diameter of the polystyrene particles being 890 nm and the mass content of solids being 10%.

### Ferrofluid

The composition may comprise a ferrofluid, i.e. a stable colloidal suspension of magnetic particles, especially of magnetic nanoparticles.

The particles, which are, for example, of the order of a few tens of nanometres in size, are dispersed in a solvent (water, oil or organic solvent), either by means of a surfactant or a dispersant, or via electrostatic interactions.

Ferrofluids are prepared, for example, by grinding ferrites or other magnetic particles until nanoparticles are obtained, which are then dispersed in a fluid containing a surfactant, which is adsorbed onto the particles and stabilizes them, or by precipitation in basic medium of a solution of metal ions.

Each particle of the ferrofluid has a magnetic moment determined by the size of the particle and by the nature of the magnetic material.

Under the action of a magnetic field, the magnetic moments of the particles align along the field lines, with the appearance of non-zero magnetization in the liquid. If the field is cancelled, there is no hysteresis and the magnetization disappears.

Beyond a threshold field value, macroscopic changes may also be brought about in the liquid, for example the appearance of peaks or a modification of the rheological properties.

The term "ferrofluid" also covers an emulsion of droplets of ferrofluid in a solvent. Each drop then contains colloidal magnetic particles in stable suspension. This affords a ferrofluid in any type of solvent. The size of the magnetic particles in suspension in the ferrofluid is, for example, between 1 nm and 10 µm, better still between 1 nm and 1 µm and even better still between 1 nm and 100 nm. The term "size" denotes the dimension given by statistical particle size distribution for half the population, referred to as D50.

Mention may be made especially of the ferrofluids sold by the company Liquids Research Ltd under the references:
- WHKS1S9 (A, B or C), which is a water-based ferrofluid comprising magnetite (Fe₃O₄), containing particles 10 nm in diameter;
- WHJS1 (A, B or C), which is a ferrofluid based on isoparaffin and magnetite (Fe₃O₄) particles 10 nm in diameter;
- BKS25_dextran, which is a water-based ferrofluid stabilized with dextran, comprising magnetite (Fe₃O₄) particles 9 nm in diameter.

### Magnetic particle and/or fibre chains

The composition may comprise particle or fibre agglomerates whose largest dimension, for example the length, is, for example, between 1 nm and 10 mm, for example between 10 nm and 5 mm or between 100 nm and 1 mm or alternatively between 0.5 µm and 3.5 mm, for example between 1 µm and 150 µm. The size denotes the dimension given by the statistical distribution for half the population, referred to as D50.

Magnetic particle chains may be obtained, for example, by assembling colloidal magnetic particles, as is described in the publications *Permanently linked monodisperse paramagnetic chains,* E.M. Furst, C. Suzuki, M. Fermigier, A.P. Gast, Langmuir, 14, 7334-7336 (1998), *Suspensions de particules magnétiques,* M. Fermigier, Y. Grasselli, Bulletin de la SFP (105) July 96, and *Flexible magnetic filaments as micromechanical sensors,* C. Goubault, P. Jop, M. Fermigier, J. Baudry, E. Bertrand, J. Bibette, Phys. Rev. Lett., 91, 26, 260802-1 to 260802-4 (2003).

These articles especially describe how to proceed in order to obtain particle chains of magnetic latices comprising a polystyrene matrix containing iron oxide grains and surface-functionalized, linked together permanently following a chemical reaction, especially covalent bonds between the surfaces of the adjacent particles; and a process is also described for obtaining chains of droplets of ferrofluid emulsion, linked together via interactions of physical nature. The length and the diameter of the permanent chains thus obtained may be controlled. Such magnetic chains constitute anisotropic magnetic objects that can be oriented and moved under the effect of a magnetic field.

The sizes of the magnetic chains may satisfy the same conditions as the magnetic fibres.

The composition containing the magnetic bodies may comprise at least one magnetic or non-magnetic nacre.

### OTHER COMPONENTS

Typically, the composition containing the magnetic bodies comprises a physiologically acceptable medium. The term "physiologically acceptable medium" denotes a non-toxic medium that may be applied to human skin, integuments or lips. The physiologically acceptable medium is generally adapted to the nature of the support onto which the composition is to be applied, and also to the form in which the composition is conditioned.

The composition may comprise ingredients other than those described above, especially at least one solvent, a fatty phase, a film-forming polymer and/or a dermatological or cosmetic active agent, especially as a function of the galenical form.

### Solvents

The composition containing the magnetic bodies may comprise at least one aqueous or organic solvent, especially a volatile organic solvent.

The first composition may advantageously comprise a volatile solvent, especially a volatile organic solvent.

For the purposes of the present invention, the term "volatile solvent" means a solvent that is liquid at room temperature, especially having a non-zero vapour pressure, at room temperature and atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg), and preferentially ranging from 1.3 Pa to 1300 Pa (0.01 to 10 mmHg).

When the composition comprises one or more organic solvents, these solvents may be present in a content ranging from 0.1% to 99% relative to the total weight of the composition concerned.

In general, the amount of solvent(s), especially organic solvent(s), will depend on the nature of the support onto which the composition is intended to be applied.

The first composition may comprise at least one volatile solvent consisting of a volatile oil.

The oil may be a silicone oil or a hydrocarbon-based oil, or may comprise a mixture of such oils.

For the purposes of the present invention, the term "silicone oil" means an oil comprising at least one silicon atom, and especially at least one Si-O group.

The term "hydrocarbon-based oil" means an oil mainly containing hydrogen and carbon atoms and possibly oxygen, nitrogen, sulfur and/or phosphorus atoms.

The volatile hydrocarbon-based oils may be chosen from hydrocarbon-based oils containing from 8 to 16 carbon atoms, and especially branched C₈-C₁₆ alkanes (also known as isoparaffins), for instance isododecane (also known as 2,2,4,4,6-pentamethylheptane), isodecane, isohexadecane and, for example, the oils sold under the trade names Isopar® or Permethyl®.

Volatile oils that may also be used include volatile silicones, for instance volatile linear or cyclic silicone oils, especially those with a viscosity ≤ 8 centistokes (8×10⁻⁶ m²/s), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the invention, mention may be made especially of dimethicones with a viscosity of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I): 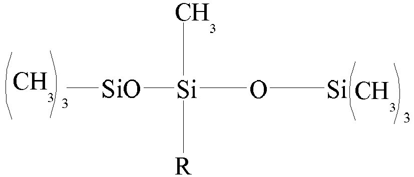 where R represents an alkyl group comprising from 2 to 4 carbon atoms, one or more hydrogen atoms of which can be replaced by a fluorine or chlorine atom.

Mention may be made, among the oils of general formula (I), of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Volatile fluoro oils such as nonafluoromethoxybutane or perfluoro-methyl-cyclopentane, and mixtures thereof, may also be used.

A composition obtained by the process according to the invention may comprise, for example, between 0.01% and 95% by weight and better still between 1% and 75% by weight of volatile oil relative to the total weight of the composition.

The composition may comprise at least one organic solvent chosen from the following list:
- ketones that are liquid at room temperature, such as methyl ethyl ketone, methyl isobutyl ketone, diisobutyl ketone, isophorone, cyclohexanone or acetone;
- alcohols that are liquid at room temperature, such as ethanol, isopropanol, diacetone alcohol, 2-butoxy-ethanol or cyclohexanol;
- glycols that are liquid at room temperature, such as ethylene glycol, propylene glycol, pentylene glycol or glycerol;
- propylene glycol ethers that are liquid at room temperature, such as propylene glycol monoethyl ether, propylene glycol monoethyl ether acetate or dipropylene glycol n-butyl ether;
- short-chain esters, especially containing from 3 to 8 carbon atoms in total, such as ethyl acetate, methyl acetate, propyl acetate, n-butyl acetate or isopentyl acetate;
- alkanes that are liquid at room temperature, such as decane, heptane, dodecane and cyclohexane.

The composition may also comprise water or a mixture of water and of hydrophilic organic solvents commonly used in cosmetics, for instance alcohols and especially linear or branched lower monoalcohols containing from 2 to 5 carbon atoms, for instance ethanol, isopropanol or n-propanol, polyols, for instance glycerol, diglycerol, propylene glycol, sorbitol or pentylene glycol, and polyethylene glycols. The first composition may also contain hydrophilic C₂ ethers and C₂-C₄ aldehydes. The water or the mixture of water and of hydrophilic organic solvents may be present in the first and/or second composition in a content ranging, for example, from 0% to 90%, especially 0. 1% to 90% by weight, preferably from 0% to 60% by weight and especially 0.1% to 60% by weight relative to the total weight of the composition.

### Fatty phase

The composition, for example when it is intended to be applied to the lips or the eyelashes, may comprise a fatty phase and especially at least one fatty phase that is liquid at room temperature (25°C) and at atmospheric pressure (760 mmHg) and/or a fatty substance that is solid at room temperature, such as waxes, pasty fatty substances and gums, and mixtures thereof. The fatty phase may also contain lipophilic organic solvents.

The composition may comprise, for example, a continuous fatty phase, which may contain less than 5% water and especially less than 1% water relative to its total weight, and in particular may be in anhydrous form.

As fatty substances that are liquid at a temperature, often referred to as "oils", mention may be made of: hydrocarbon-based plant oils such as liquid triglycerides of fatty acids of 4 to 10 carbon atoms such as heptanoic or octanoic acid triglycerides, or alternatively sunflower oil, corn oil, soybean oil, grapeseed oil, sesame seed oil, apricot oil, macadamia oil, castor oil, avocado oil, caprylic/capric acid triglycerides, jojoba oil, shea butter oil, lanolin oil or acetylated lanolin oil; linear or branched hydrocarbons, of mineral or synthetic origin, such as liquid paraffins and derivatives thereof, petroleum jelly, polydecenes or hydrogenated polyisobutene such as Parleam; synthetic esters and ethers, especially of fatty acids, for instance Purcellin oil, isopropyl myristate, 2-ethylhexyl palmitate, 2-octyldodecyl stearate, 2-octyldodecyl erucate or isostearyl isostearate; hydroxylated esters such as isostearyl lactate, octyl hydroxystearate, octyldodecyl hydroxystearate, diisostearyl malate, triisocetyl citrate, and fatty alcohol heptanoates, octanoates or decanoates; isononyl isononanoate, isopropyl lanolate, tridecyl trimellitate or diisostearyl malate; polyol esters such as propylene glycol dioctanoate, neopentyl glycol diheptanoate, diethylene glycol diisononanoate; and pentaerythritol esters; fatty alcohols containing from 12 to 26 carbon atoms, such as octyldodecanol, 2-butyloctanol, 2-hexyldecanol, 2-undecylpentadecanol or oleyl alcohol; partially hydrocarbon-based and/or silicone-based fluoro oils, silicone oils such as volatile or non-volatile, linear or cyclic polydimethylsiloxanes (PDMS), which are liquid or pasty at room temperature, such as cyclomethicones, dimethicones, optionally comprising a phenyl group, such as phenyl trimethicones, phenyltrimethylsiloxydiphenylsiloxanes, diphenylmethyldimethyltrisiloxanes, diphenyl dimethicones, phenyl dimethicones and polymethylphenylsiloxanes; and mixtures thereof. The oil(s) may be present in a content ranging from 0.01% to 90% by weight and better still from 0.1% to 85% by weight relative to the total weight of the composition.

The presence of an oily phase may impart gloss and may have, for example, a refractive index of between 1.47 and 1.51 and better still between 1.48 and 1.50. The refractive index is measured at room temperature (25°C) using a refractometer.

The composition may comprise at least one agent for structuring the liquid fatty phase (formed by the volatile or non-volatile oils and/or organic solvents described above) chosen from waxes, semi-crystalline polymers and lipophilic gelling agents, and mixtures thereof.

The pasty fatty substances are generally hydrocarbon-based compounds with a melting point of between 25 and 60°C and preferably between 30 and 45°C and/or a hardness between 0.001 and 0.5 MPa and preferably between 0.005 and 0.4 MPa, for instance lanolins and derivatives thereof.

The waxes may be solid at room temperature (25°C), with a reversible solid/liquid change of state, having a melting point of greater than 30°C, which may be up to 200°C, a hardness of greater than 0.5 MPa and having in the solid state an anisotropic crystal organization. In particular, the waxes may have a melting point of greater than 25°C and better still greater than 45°C. The waxes may be hydrocarbon-based waxes, fluoro waxes and/or silicone waxes and may be of plant, mineral, animal and/or synthetic origin. As waxes that may be used, mention may be made of beeswax, carnauba wax or candelilla wax, paraffin, microcrystalline waxes, ceresin or ozokerite; synthetic waxes such as polyethylene waxes or Fischer-Tropsch waxes, silicone waxes such as alkyl or alkoxy dimethicones containing from 16 to 45 carbon atoms. The composition may contain from 0 to 50% by weight or even from 1% to 30% by weight of waxes relative to the total weight of the composition.

The gums that may be used are generally polydimethylsiloxanes (PDMS) of high molecular weight or cellulose gums or polysaccharides.

### Film-forming polymers

The composition may comprise, for example, a film-forming polymer, especially in the case of a mascara, a nail varnish or a foundation. The term "film-forming polymer" denotes a polymer that is capable of forming, by itself or in the presence of a film-forming auxiliary agent, a continuous film that adheres to a support, especially to keratin materials.

Among the film-forming polymers that may be used in the composition according to the invention, mention may be made, inter alia, of synthetic polymers, of free-radical type or of polycondensate type, polymers of natural origin, such as nitrocellulose or cellulose esters, and mixtures thereof.

The film-forming polymers of free-radical type may be, in particular, vinyl polymers or copolymers, in particular acrylic polymers.

The vinyl film-forming polymers may result from the polymerization of ethylenically unsaturated monomers containing at least one acid group and/or esters of these acidic monomers and/or amides of these acidic monomers, for instance α,β-ethylenic unsaturated carboxylic acids such as acrylic acid, methacrylic acid, crotonic acid, maleic acid or itaconic acid.

The vinyl film-forming polymers may also result from the homopolymerization or copolymerization of monomers chosen from vinyl esters such as vinyl acetate, vinyl neododecanoate, vinyl pivalate, vinyl benzoate and vinyl t-butylbenzoate, and styrene monomers such as styrene and alpha-methylstyrene.

Among the film-forming polycondensates that may be mentioned are polyesters, polyester-amides, polyamides and polyureas, this list not being limiting.

The polymers of natural origin, optionally modified, may be chosen from shellac resin, sandarac gum, dammar resin, elemi gum, copal resin, cellulose-based polymers, such as nitrocellulose, ethylcellulose or nitrocellulose esters chosen, for example, from cellulose acetate, cellulose acetobutyrate and cellulose acetopropionate, and mixtures thereof.

The film-forming polymer may be in the form of solid particles in aqueous or oily dispersion, which is generally known as a latex or pseudolatex. The film-forming polymer may comprise one or more stable dispersions of generally spherical polymer particles of one or more polymers, in a physiologically acceptable liquid fatty phase. These dispersions are generally known as NADs (non-aqueous dispersions) of polymer, as opposed to latices, which are aqueous polymer dispersions. These dispersions may especially be in the form of polymer nanoparticles in stable dispersion in the said fatty phase. The nanoparticles are preferably between 5 and 600 nm in size. Techniques for preparing these dispersions are well known to those skilled in the art.

Aqueous dispersions of film-forming polymer that may be used include the acrylic dispersions sold under the names Neocryl XK-90® , Neocryl A-1070® , Neocryl A-1090® , Neocryl BT-62® , Neocryl A-1079® , Neocryl A-523® by the company Avecia-Neoresins, Dow Latex 432® by the company Dow Chemical, Daitosol 5000 AD® by the company Daito Kasei Kogyo; or else the aqueous polyurethane dispersions sold under the names Neorez R-981® , Neorez R-974® by the company Avecia-Neoresins, Avalure UR-405® , Avalure UR-410® , Avalure UR-425® , Avalure UR-450® , Sancure 875® , Sancure 861® , Sancure 878® , Sancure 2060® by the company Goodrich, Impranil 85® by the company Bayer, Aquamere H-1511® by the company Hydromer; the sulfopolyesters sold under the brand name Eastman AQ by the company Eastman Chemical Products.

### Block film-forming polymer

According to one embodiment of the invention, the composition comprises at least one film-forming polymer that is a film-forming linear block ethylenic polymer. This polymer preferably comprises at least a first block and at least a second block with different glass transition temperatures (Tg), the said first and second blocks being linked together via an intermediate block comprising at least one constituent monomer of the first block and at least one constituent monomer of the second block,

Advantageously, the first and second blocks of the block polymer are mutually incompatible.

Such polymers are described, for example, in document EP 1 411 069 or WO 04/028 488.

The composition containing the magnetic bodies may comprise at least one magnetic or non-magnetic filler.

### Fillers

The term "fillers" denotes particles of any form that are insoluble in the medium of the composition, irrespective of the temperature at which the composition is manufactured. A filler may serve especially to modify the rheology or the texture of the composition. The nature and amount of the particles may depend on the desired mechanical properties and textures.

Examples of fillers that may be mentioned include, inter alia, talc, mica, silica, kaolin, sericite, polyamide powder, polyolefin powder, for example polyethylene powder, polytetrafluoroethylene powder, polymethyl methacrylate powder or polyurethane powder, starch powders and silicone resin beads.

The fillers may be intended to create, inter alia, a soft-focus effect, especially in the case of foundation, so as to hide skin imperfections.

The composition containing the magnetic bodies may also comprise an auxiliary film-forming agent that promotes the formation of a film with the film-forming polymer.

### Active agents

The composition may comprise at least one cosmetic or dermatological active agent. As cosmetic, dermatological, hygiene or pharmaceutical active agents that may be used in the compositions obtained by the process of the invention, mention may be made of moisturizers (polyols, for instance glycerol), vitamins (C, A, E, F, B or PP), essential fatty acids, essential oils, ceramides, sphingolipids, sunscreens that are liposoluble or in the form of nanoparticles, and specific skin-treating active agents (protective agents, antibacterial agents, anti-wrinkle agents, etc.), and self-tanning agents. These active agents may be used, for example, in concentrations of from 0 to 20% and especially from 0.001% to 15% relative to the total weight of the composition.

The composition may also contain ingredients commonly used in cosmetics, for instance thickeners, surfactants, trace elements, moisturizers, softeners, sequestrants, fragrances, acidifying or basifying agents, preserving agents, antioxidants, UV-screening agents and dyes, or mixtures thereof.

Depending on the intended type of application, the composition obtained by the process according to the invention may comprise the constituents conventionally used in the fields under consideration, which are present in an amount that is suitable for the intended galenical form.

Needless to say, a person skilled in the art will take care to select this or these optional additional compound(s), and/or the amount thereof, such that the advantageous properties of the composition obtained by the process according to the invention are not, or are not substantially, adversely affected by the envisaged addition.

The examples of compositions below are given as non-limiting illustrations.

The assembly may be understood more clearly on reading the detailed description that follows, of non-limiting examples of the use thereof, and on inspection of the attached drawings, in which:
- Figure 1 shows schematically an example of a magnetic device before switching it on to produce a composition obtained by the process according to the invention,
- Figures 2 to 5 show schematically, in partial axial cross section, the magnetic device of Figure 1 after operating it, various compositions being produced,
- Figures 6 to 13 show in transverse cross section various compositions obtained by the process according to the invention,
- Figures 14 to 19 show in longitudinal cross section various compositions obtained by the process according to the invention,
- Figure 20 shows another conditioning of a composition obtained by the process according to the invention.

A lipstick, an antiperspirant and a deodorant stick are prepared. The following commercial compositions are used:

| | |
|---|---|
| For the lipstick | LANCÔME ROUGE ABSOLU |
| For the antiperspirant | STICK INVISIBLE GARNIER MINERAL HOMME |
| For the deodorant stick | MENNEN STICK LARGE CONTROL H24 GREEN |

The commercial compositions are brought to a temperature ranging from 75 to 120°C. 1% by weight, relative to the total weight of the composition, of magnetic nacres is added. Each composition 30 is cast, while hot and in the fluid state, in a stick mould or in a cupel.

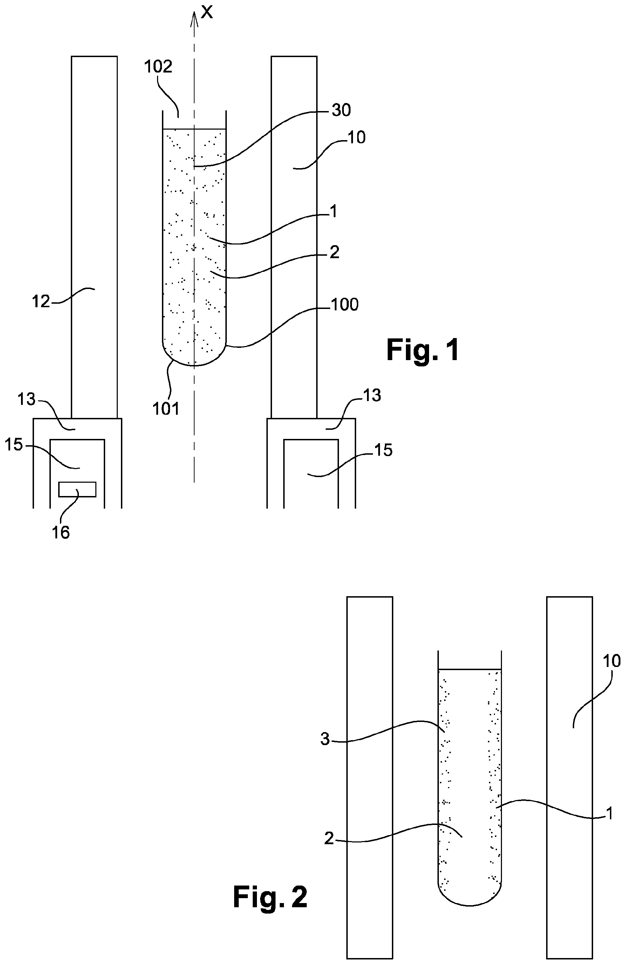

Figure 1 shows a composition 30 comprising magnetic bodies 1 cast in a cylindrical stick mould 100. The longitudinal axis X passes through the cylinder 100.

The magnetic bodies 1 are non-randomly distributed in the composition 30.

The composition 30 is contained in the hollow cylinder 100. This cylinder is closed at one end by a base 101 and open at the opposite end 102. This cylinder is made of silicone. It may also be made from other materials such as those constituting cosmetic conditioning articles or various casting moulds: polypropylene, polyethylene, PVC, aluminium, etc.

The mould 100 is placed at the centre of a magnetic device 10 which can generate a magnetic field that is useful for modifying the appearance of the composition 30 without coming into contact with it.

In the example under consideration, the magnetic device 10 comprises a permanent magnet 12 supported by a holding member 13 whose longitudinal axis is parallel to the axis X, the polar axis of the magnet 12 being substantially perpendicular to the axis X.

In the example under consideration, the magnetic device 10 is arranged to generate a rotating magnetic field and comprises a concealed motor, housed in a case 15, to drive the holding member 13 in rotation about the axis X.

A switch 16 is present on the case 15 to enable the user to switch on the motor and drive in rotation the holding member 13 with the magnet 12.

The strength of the magnet 12 is advantageously between 0.2 and 1.4 T.

It is preferably placed a distance from the composition 30 of between 0.01 and 3 mm.

More preferentially, the magnetic field is created in the device over a period of between 1 and 30 seconds.

In one variant embodiment not shown, the rotating magnetic field is generated by a plurality of solenoids that are powered sequentially so as to generate a rotating field.

When the device is on, the magnetic particles 1 contained in the composition 30 become aligned with the field lines of the magnet 12 and change their orientation, which leads to a change in the appearance of the composition 30.

The user may select the parameters for applying the magnetic field as a function of the result she desires to obtain.

The pattern obtained may be, for example, continuous as in Figure 2, in which the particles 1 are grouped together at the periphery of the stick. They form a hollow cylinder enveloping a core that is depleted in magnetic bodies.

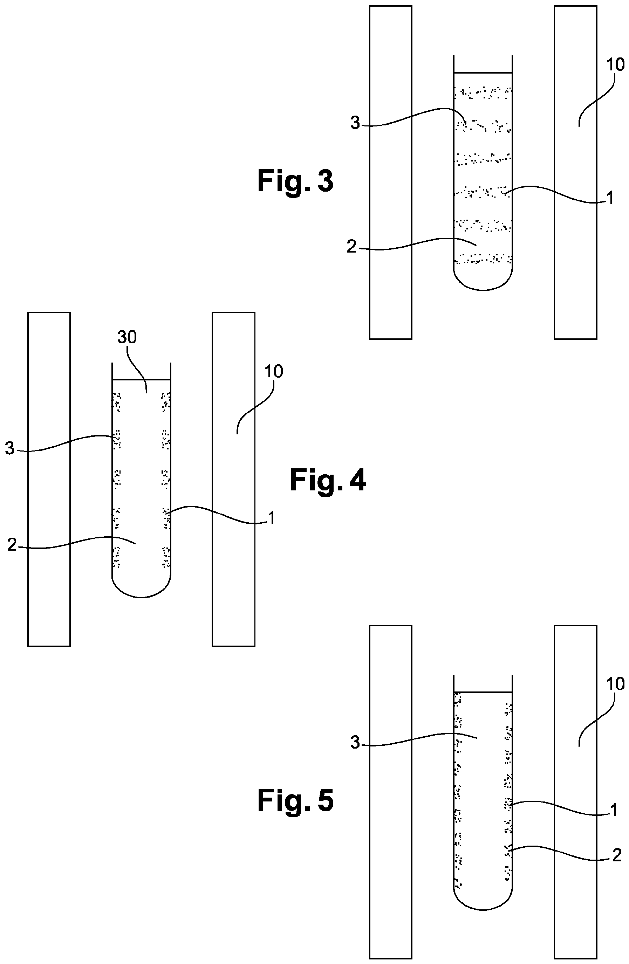

The pattern obtained may, for example, be discontinuous, as in Figures 3 to 5, in which the particles 1 are grouped together in lumps, to form repeating patterns that are distinct from each other.

In this case, the magnetic device 10 is, for example, in the form of a structure charged with magnetized particles, creating an alternation of N and S holes, which makes it possible to form repeating patterns, for example strips, lines or traces.

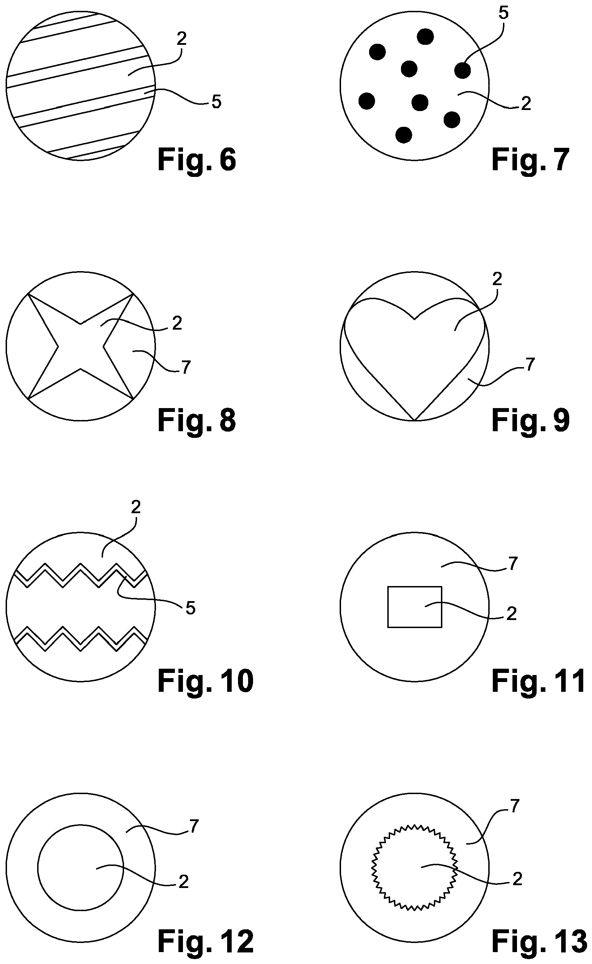

Figures 6 to 13 show transverse cross sections of sticks obtained by the process according to the invention. The patterns obtained may form isolated regions 5 that are distinguished from the regions 2 depleted in magnetic particles. These patterns 5 may, for example, form stripes, circles or zigzags.

It may also be envisaged to form a heart-shaped, square or circular transverse cross section with or without teeth. However, other patterns may be made.

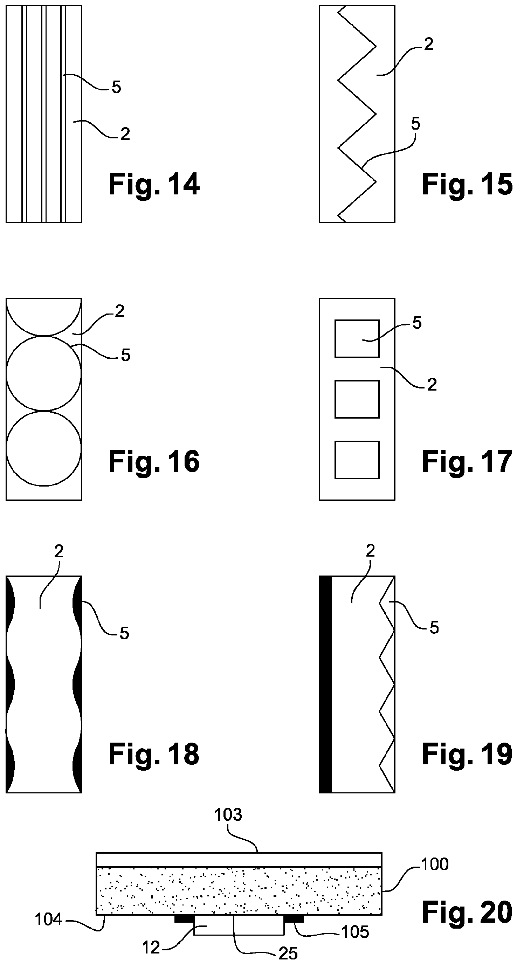

Figures 14 to 19 show transverse cross sections of sticks obtained by the process according to the invention. The patterns 5 may appear in the form of lines, zigzags, curves, parallelepipeds, decorative cords or strips in transverse cross section. However, other patterns may be made.

Another device for making a composition is shown in Figure 20. In this embodiment, the composition has been cast in a cupel 100 forming a case. This case consists of a base body 104 and a lid 103 articulated thereon.

The base body 104 comprises a compartment housing the composition consisting, in the illustrated example, of a foundation in pasty form.

The base body 104 also comprises a housing 105 arranged to receive at least one magnet 12.

This magnet 12 may have, for example, an adhesive face 25 or any other assembly means for fixing it to the housing 105. This magnet may be removed after manufacturing the composition or may remain attached to the body 104 depending on the pattern created in the composition 30.

The magnet 12 may, of course, be located on the lid 103 or on the side walls of the cupel 100.

Similarly, instead of being positioned in a cupel, the magnet may be positioned on the walls of a deodorant tube or of a lipstick.

## Claims

1. Process for manufacturing a solid cosmetic composition cast as a stick the said composition having an outer surface, and containing one or more magnetic bodies (1) of non-zero magnetic susceptibility, the magnetic bodies (1) are on at least part of the said outer surface non-randomly oriented in the solid composition so as to form on the said outer surface one or more pattern(s) (5), the process is **characterized in that** it comprises the following steps:
- preparing a fluid cosmetic composition comprising one or more magnetic bodies (1) of non-zero magnetic susceptibility,
- casting the cosmetic composition while hot as a stick,
- at least partially exposing the composition to a magnetic field (10), so as to modify the orientation of and/or at least partly to move the magnetic bodies (1) when the composition is fluid, in order to distribute them non-randomly in the solid composition.

2. Process according to Claim 1, in which the magnetic field is applied so as to form on the outer surface of the stick one or more pattern(s) (5).

3. Process according to any of the preceding claims, in which the cosmetic composition is cast at a temperature of between 75°C and 120°C and more preferentially between 85°C and 110°C.

## Patentansprüche

1. Verfahren zur Herstellung einer festen kosmetischen Zusammensetzung, die in Form eines Sticks gegossen ist, wobei die Zusammensetzung eine äußere Oberfläche aufweist und einen oder mehrere magnetische Körper (1) mit einer von null verschiedenen magnetischen Suszeptibilität enthält, wobei die magnetischen Körper (1) auf mindestens einem Teil der äußeren Oberfläche in der festen Zusammensetzung nicht zufällig orientiert sind und auf der äußeren Oberfläche ein oder mehrere Muster (5) bilden, wobei das Verfahren **dadurch gekennzeichnet ist, dass** es die folgenden Schritte umfasst:
- Herstellen einer fließfähigen kosmetischen Zusammensetzung, die einen oder mehrere magnetische Körper (1) mit einer von null verschiedenen magnetischen Suszeptibilität umfasst,
- Gießen der kosmetischen Zusammensetzung in der Hitze als Stick,
- mindestens teilweises Einwirkenlassen eines Magnetfelds (10) auf die Zusammensetzung zum Modifizieren der Orientierung und/oder mindestens teilweise Bewegen der magnetischen Körper (1), wenn die Zusammensetzung fließfähig ist, um sie in der festen Zusammensetzung nicht zufällig zu verteilen.

2. Verfahren nach Anspruch 1, bei dem das Magnetfeld zur Bildung eines oder mehrerer Muster (5) auf der äußeren Oberfläche des Sticks angelegt wird.

3. Verfahren nach einem der vorhergehenden Ansprüche, bei dem die kosmetische Zusammensetzung bei einer Temperatur zwischen 75 °C und 120 °C und weiter bevorzugt zwischen 85 °C und 110 °C gegossen wird.

## Revendications

1. Procédé de fabrication d'une composition cosmétique solide coulée en stick, ladite composition présentant une surface extérieure, et contenant un ou plusieurs corps magnétiques (1) présentant une susceptibilité magnétique non nulle, les corps magnétiques (1) sont sur au moins une partie de ladite surface extérieure orientés de manière non aléatoire dans la composition solide de manière à former sur ladite surface extérieure un ou plusieurs motif (s) (5), le procédé étant **caractérisé en ce qu'**il comporte les étapes suivantes :
- préparer une composition cosmétique fluide comportant un ou plusieurs corps magnétiques (1) présentant une susceptibilité magnétique non nulle,
- couler à chaud la composition cosmétique en stick,
- exposer au moins partiellement la composition à un champ magnétique (10), de manière à modifier l'orientation et/ou déplacer au moins une partie des corps magnétiques (1) lorsque la composition est fluide, afin de les répartir de manière non aléatoire dans la composition solide.

2. Procédé selon la revendication 1, dans lequel le champ magnétique est appliqué de manière à former sur la surface extérieure du stick un ou plusieurs motif(s) (5).

3. Procédé selon l'une quelconque des revendications précédentes, dans lequel la composition cosmétique est coulée à une température comprise entre 75 °C et 120 °C, et plus préférentiellement entre 85 °C et 110 °C.
